(19) Europäisches Patentamt
European Patent Office
Office européen des brevets

(11) Publication number: 0 031 252
B1

(12) # EUROPEAN PATENT SPECIFICATION

(45) Date of publication of patent specification: 25.01.84

(21) Application number: 80304620.0

(22) Date of filing: 19.12.80

(51) Int. Cl.[3]: C 07 C 67/08, C 07 C 67/04, C 07 C 67/26, C 07 C 69/14, C 07 C 69/24, C 07 C 41/06, C 07 C 85/20, C 07 C 2/70, B 01 J 21/00, B 01 J 39/00, C 07 C 43/04

(54) **Proton-catalysed reactions in which water is not a stoichiometric reactant catalysed by metal cation-exchanged layered clays.**

(30) Priority: 22.12.79 GB 7944315
17.05.80 GB 8016384
05.07.80 GB 8022101
09.08.80 GB 8026028

(43) Date of publication of application:
01.07.81 Bulletin 81/26

(45) Publication of the grant of the patent:
25.01.84 Bulletin 84/4

(84) Designated Contracting States:
BE DE FR GB IT NL

(56) References cited:
FR - A - 710 846
FR - A - 1 365 733
FR - A - 1 482 271
FR - A - 1 583 593

(73) Proprietor: The British Petroleum Company p.l.c.
Britannic House Moor Lane
London EC2Y 9BU (GB)

(72) Inventor: Ballantine, James Archibald
University College of Swansea Singleton Park
Swansea SA2 8PP (GB)
Inventor: Purnell, John Howard
University College of Swansea Singleton Park
Swansea SA2 8PP (GB)
Inventor: Thomas, John Meurig
University of Cambridge Lensfield Road
Cambridge CB2 1EP (GB)

(74) Representative: Harry, John et al,
BP INTERNATIONAL LIMITED Patents and
Licensing Division Chertsey Road
Sunbury-on-Thames Middlesex TW16 7LN (GB)

EP 0 031 252 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Proton-catalysed reactions in which water is not a stoichiometric reactant catalysed by metal cation-exchanged layered clays

The present invention relates generally to proton-catalysed organic reactions in which interlamellar water is not one of the stoichiometric reactants and in particular to the use of a cation-exchangeable layered clay in which the exchangeable cation is a metal cation as catalysts in such reactions.

Many different types of organic reaction are catalysed by protons or, to give them another name, hydrogen ions. Typical of such reactions are olefin hydration in which the product is an alcohol, esterification of an alcohol with an acid in which the product is an ester and the decomposition of organic hydroperoxides, e.g. cumene hydroperoxide in which the products are phenol and acetone. Generally the protons are provided by the dissociation of a strong mineral acid or a strong organic acid. Thus sulphuric acid and para-toluene sulphonic acid have been used extensively as catalysts in the industrial production of esters, and phosphoric acid, usually supported on silica, is a catalyst commonly employed in the commercial production of ethanol.

French Patents Nos 1365733 and 1583593 describe the use of solid metal cation-exchanged resins in the production of esters by the reaction of an alcohol with a carboxylic acid.

In the Journal of Physical Chemistry, Volume 44, No. 2, February, 1940, pp 180 to 184, there is disclosed the preparation of an acid bentonite by electrodialyzing a 4 per cent suspension of Wyoming bentonite in a cell of the Mattson type until the catholyte liquor is no longer alkaline, followed by ion-exchange of the acid bentonite with an aqueous solution of a metal salt to produce a metal cation-exchanged bentonite and the use of the metal cation-exchanged bentonite so-prepared as catalyst in the decomposition of hydrogen peroxide.

Thereafter in the Journal of Catalysts 58, 238—252 (1979) Adams et al disclosed that metal cation-exchanged water-intercalated clays such as metal cation-exchanged water-intercalated montmorillonites will convert alkenes to the corresponding bis-sec-alkyl ethers. In some circumstances 100% conversion of usable intercalated water to ether was achieved but it was not possible to make the reaction self-sustaining by the addition of water to the reaction.

We have now found that cation-exchangeable layered clays in which the exchangeable cation is a metal cation catalyse organic reactions which are catalysed by protons in which intercalated water is not one of the stoichiometric reactants. An example of such a reaction is the direct reaction of olefins with acids to make esters.

Accordingly the present invention provides a process for carrying out a proton-catalysed organic reaction in which intercalated water is not one of the stoichiometric reactants employing as catalyst a metal cation-exchangeable material characterised in that as the cation-exchangeable material there is used a layered clay in which the exchangeable cation is a metal cation.

A layered clay within the context of the present specification is a clay having a lamellar structure with interlamellar spaces disposed between the lamellar layers. Typical of such clays is montmorillonite which has an idealised stoichiometric composition corresponding to $Na_{0.67}[Al_{3.33}Mg_{0.67}](Si_8)O_{20}(OH)_4$. Structurally it comprises a central octahedral co-ordination layer containing aluminium and magnesium oxides and hydroxides sandwiched between two tetrahedral co-ordination layers containing silicon oxide. Normally in nature cations are present to compensate for the charge imbalance caused by isomorphous substitution of $Mg^{2+}$ for $Al^{3+}$ in the octahedral layer, and/or $Al^{3+}$ or other ions for $Si^{4+}$ in the tetrahedral layers. The octahedral and tetrahedral regions are tightly bound together to form a lamellar layer. The space between the lamellar layers, i.e. the interlamellar space, is normally occupied by exchangeable $Ca^{2+}$ or $Na^+$ ions. The distance between the interlamellar layers can be substantially increased by absorption of a variety of polar molecules such as water, ethylene glycol, amines etc., which enter the interlamellar space and in doing so push apart the lamellar layers. The interlamellar spaces tend to collapse when the molecules occupying the space are removed, for example by heating the clay at a high temperature. Both natural and synthetic clays having a layered structure are well known and may be used in the process of the invention both before and after exchange of the metal cations normally associated therewith with other metal cations. Besides montmorillonites such as bentonite and Fullers Earths, other types of suitable clays include hectorites, beidellites, vermiculites and nontronite. A preferred clay is a bentonite, such as Wyoming bentonite.

As hereinbefore described the clays in their natural state normally contain exchangeable sodium or calcium ions in the interlamellar space. Such clays have some catalytic activity in the process of the present invention. In order to bestow increased catalytic activity on the clay it is necessary to exchange some or all of the exchangeable metal cations with cations of one or more other suitable metals. Examples of suitable metals include chromium, aluminium, cobalt, nickel, iron, copper and vanadium, of which chromium and aluminium are preferred.

Ion-exchange itself is a technique well known in the art. Although any of the variants of that technique may be used in the preparation of catalysts useful in the process of the present invention the metal cation-exchanged clay is preferably prepared by exchanging the sodium or calcium or other exchangeable cations in a natural clay with an aqueous solution of a metal salt rather than by exchanging with ammonium ions from an aqueous solution of an ammonium compound to form the

ammonium ion-exchanged clay, followed by calcination of the ammonium ion-exchanged clay to form the hydrogen-ion exchanged clay and subsequent exchange with an aqueous solution of a metal salt to produce the metal cation-exchanged clay. This preference arises from the desirability of avoiding excessively high temperatures, such as those obtaining during calcination because their use tends to collapse the lamellar structure of the clay and lead to inactive catalysts.

Techniques for separating the metal cation-exchanged clay from the ion-exchange media and excess ions are well known. Any suitable solid/liquid separation procedure can be used. Decantation or centrifugation are two preferred methods for solid/liquid separation.

After exchange the metal cation-exchanged clay is preferably washed until all extraneous metal cations are removed and dried, suitably at a temperature which does not result in collapse of the interlamellar space. The temperature will depend to some extent on the clay selected but for certain types of exchanged bentonites temperatures up to 220°C, preferably from 80 to 220°C are suitable.

Cation-exchangeable layered clays in which the exchangeable cation is a metal cation may be used as catalysts in all organic reactions catalysed by protons in which intercalated water is not one of the stoichiometric reactants. Advantages arising from their use are that they can be readily separated from the reaction mixture which renders them useful in continuous processes, and they are less corrosive than the strong acids conventionally employed. An advantage of clays over ion-exchange resins is that they are very much cheaper. We have found the clays to be particularly useful catalysts in certain specific organic reactions, such as the production of esters by the reaction of an olefin with a carboxylic acid and the production of ethers by reaction of an alcohol and certain alkenes.

In a particular aspect therefore the present invention provides a process for the production of esters which process comprises reacting one or more olefines with one or more carboxylic acids in the presence as catalyst of a cation-exchangeable layered clay in which the exchangeable cation is a metal cation under reaction conditions which result in the formation of an ester.

With regard to the olefin reactant any suitable olefin may be employed. Suitable olefins include ethylene, propylene, butenes, pentenes and hexenes, diolefins such as butadiene and cyclic olefins such as cyclohexene. Mixtures of olefins such as those commonly encountered in refinery streams may also be used if so desired. The amount of olefin employed may be greater or less than the stoichiometric amount required to react completely with the acid.

Both aromatic and aliphatic carboxylic acids may be used. Suitable aliphatic acids include formic, acetic, propionic and butyric acids. Of the aromatic acids phthalic acids, especially orthophthalic acid, may be employed. Mixtures of acids may also be used.

Preferably the olefin is ethylene, the carboxylic acid is acetic acid and the ester produced is ethyl acetate.

A preferred metal cation-exchanged layered clay for use in this process is a chromium or aluminium ion-exchanged sodium bentonite. The catalyst may suitably be activated before use by heating in air at a temperature up to 220°C, preferably from 80 to 220°C.

The process may be carried out in the liquid phase or in the vapour phase, preferably in the liquid phase. Reaction conditions which result in the formation of esters will depend on whether the process is carried out in the liquid or the vapour phase and to some extent on the nature of the reactants.

In the liquid phase the pressure is suitably that pressure which maintains a liquid phase at the reaction temperature. In the case of olefins with suitably high boiling points, e.g. hexene-1, the reaction may for example be conveniently carried out at the reflux temperature of the reactants and under atmospheric pressure, or at higher temperatures and pressures if so desired. In the case of ethylene, for example, initial pressures in the range 25 to 150 bar may suitably be employed. Generally the temperature may suitably be in the range 100 to 300°C, preferably 150 to 250°C. Solvents may be employed if desired. Suitable solvents include hydrocarbons, e.g. alkanes such as ethane, hexane and octane.

In the vapour phase the conditions must be chosen so that the reactants do not liquefy; for example acetic acid must be fed at atmospheric or slightly higher pressure otherwise it would liquefy at higher pressures. Generally the temperature will suitably be in the range 120 to 180°C, preferably 140 to 160°C. The residence time, which is defined as:—

$$\frac{\text{Volume of catalyst in mls}}{\text{Vapour flow rate (in mls/sec at NTP)}}$$

may suitably be in the range 10 to 60 secs, preferably 20 to 40 secs.

The process may be carried out batchwise or continuously, preferably continuously. The batchwise liquid phase production of ethyl acetate, for example, may conveniently be carried out by charging acetic acid and catalyst to an autoclave, pressurising the autoclave with ethylene and maintaining the autoclave at the reaction temperature. The reaction time should not be unduly protracted otherwise the selectivity for the conversion of acetic acid to ethyl acetate may be adversely affected. Thus at an approximately 2:1 molar ratio of ethylene to acetic acid, an initial ethylene pressure of 55 bar and a

temperature of 200°C, the reaction time should preferably not exceed 5 hours. At the completion of the reaction the catalyst may be recovered from the product, suitably by filtration, centrifugation or decantation and the product worked up in known manner to recover ethyl acetate therefrom. The catalyst may thereafter be re-used in a further batch reaction with or without intervening treatment.

The invention also provides a process for the production of ethers which process comprises reacting at a temperature above 100°C and in the presence of a cation-exchangeable layered clay in which the exchangeable cation is a metal cation one or more alcohols with one or more olefins having the structure:—

$$R^1CH = CHR^2$$

wherein $R^1$ and $R^2$ are independently H, alkyl or aryl or are bonded together to form a ring.

Suitable alcohols include methanol, ethanol, propanols, butanols, pentanols and hexanols.

With regard to the olefin any suitable olefin may be employed. Suitable olefins include ethylene, propylene, butenes, pentenes and hexenes, diolefins such as butadiene and cyclic olefins such as cyclohexene. Preferably the olefin is a $C_3$ to $C_6$ olefin. Mixtures of olefins such as those commonly encountered in refinery streams may also be used if so desired. The amount of olefin employed may be greater or less than the stoichiometric amount required to react completely with the alcohol.

A preferred catalyst for use in this process is an aluminium or chromium ion-exchanged sodium bentonite. The catalyst may suitably be activated before use by heating in air at a temperature up to 200°C, preferably from 80 to 160°C.

The process may be carried out in the liquid phase or in the vapour phase, preferably in the liquid phase. Reaction conditions which result in the formation of an ether will depend on whether the process is carried out in the liquid or the vapour phase and to some extent on the nature of the reactants.

In the liquid phase the pressure is suitably that pressure which maintains a liquid phase at the reaction temperature. In the case of olefins with suitably high boiling points the reaction may for example be conveniently carried out at the reflux temperature of the reactants and under atmospheric pressure, or at higher temperatures and pressures if so desired. Generally the temperature may be between 100 and 300°C, preferably 150 to 250°C. The particular temperature employed within the aforesaid ranges will depend upon the nature of the olefins. Solvents may be employed if so desired. Suitable solvents include hydrocarbons, e.g. alkanes such as ethane, hexane and octane.

The process may be carried out batchwise or continuously, preferably continuously.

Other proton-catalysed reactions of interest which are catalysed by cation-exchanged layered clays in which the exchangeable cation is a metal cation include:—

(i) the production of ethers by reacting at elevated temperature and a pressure such that the reactants are maintained in the liquid phase one or more primary or secondary aliphatic alcohols or polyols in the presence of a metal cation-exchangeable layered clay in which the exchangeable cation is a metal cation.

The ethers are believed to be produced by condensation of two alcohol functions accompanied by elimination of water.

With regard to the primary aliphatic alcohol reactant suitable alcohols include methanol, ethanol, propan-1-ol, butan-1-ol, pentan-1-ol, hexan-1-ol, heptan-1-ol and octan-1-ol. The principal ether in the product resulting from the reaction of a primary aliphatic alcohol in the presence of the lamellar clays is the corresponding 1,1,-ether, though the corresponding 1,2-ether, may also be formed. Alkenes and alkene dimers may also be formed. Generally the proportion of alkene in the product increases as the carbon number of the reactant alcohol increases.

With regard to the secondary aliphatic alcohol reactant suitable alcohols include straight-chain alcohols such as propan-2-ol, butan-2-ol, pentan-2-ol, hexan-2-ol and hexan-3-ol, and cyclohexanol, of which propan-2-ol and butan-2-ol are preferred. The ethers predominating in the product resulting from the reaction of alkan-2-ol and alkan-3-ols are the 2,2,- and 3,3- ethers respectively. Alkenes and alkene dimers are also formed.

The reactant may also be a polyol such as an alkylene glycol. A suitable alkylene glycol is ethylene glycol which produces a mixture of dioxan, and di-, tri-, tetra- etc., ethylene glycol. A preferred alkylene glycol is diethylene glycol which produces dioxan in high conversions in the presence of the lamellar clay. Additionally mixtures of alcohols and/or polyols may be used if so desired.

The elevated temperature may suitably be in the range from 100 to 300°C, preferably from 150 to 225°C.

(ii) the production of ethers by reacting one or more epoxides in the presence of a cation-exchangeable layered clay in which the exchangeable cation is a metal cation.

Thus for example reaction of ethylene oxide yields 1,4-dioxane and 2 methyl-1,3-dioxan. Other epoxides produce cyclic ethers but alpha, beta-unsaturated aldehydes may also be formed. The proportion of the unsaturated aldehyde generally tends to increase with the carbon number of the epoxide.

(iii) the formation of esters by reacting one or more epoxides with one or more carboxylic acids in

the presence of a cation-exchangeable layered clay in which the exchangeable cation is a metal cation as catalyst.

Thus for example ethylene glycol diacetate and 2-hydroxy ethyl acetate can be obtained by reaction of ethylene oxide and acetic acid.

(iv) the formation of ethers by reacting one or more epoxides with one or more alcohols, polyols or polysaccharides in the presence of a cation-exchangeable layered clay in which the exchangeable cation is a metal cation.

Thus for example 2-ethoxy ethanol, diethylene glycol monoethyl ether, ethylene glycol diethyl ether and diethylene glycol diethyl ether can be obtained by reaction of ethylene oxide and ethanol.

(v) the formation of alpha, beta-unsaturated aldehydes by reacting aldehydes with a cation exchangeable layered clay in which the exchangeable cation is a metal cation.

Thus for example but-2-en-1-al can be obtained by reaction of acetaldehyde.

(vi) the production of a secondary or a tertiary amine by reacting at elevated temperature a primary or a secondary amine having a methylene group adjacent to an amino group in the presence as catalyst of a metal cation-exchangeable layered clay, reaction of a primary amine resulting in the formation of a secondary amine and reaction of a secondary amine resulting in the formation of a tertiary amine.

Provided there is a methylene group adjacent to the amino group the primary amine may be an aliphatic, cycloaliphatic or aromatic amine. Examples of suitable primary amines which may be employed in the process of the invention include hexan-1-amine, benzylamine and cyclohexylamine. The products are believed to be formed by condensation of two molecules of the primary amine accompanied by the elimination of a single molecule of ammonia. Thus, for example, in the case of benzylamine the reaction may be represented as follows:—

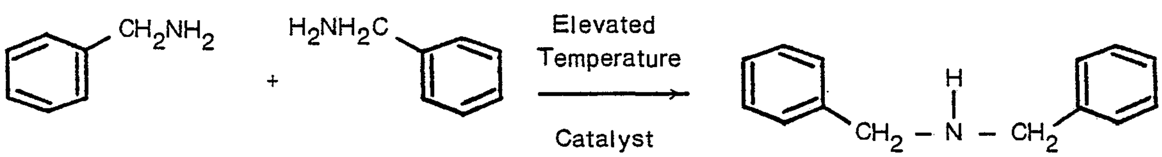


The secondary amine having a methylene group adjacent to an amino group is preferably a heterocyclic compound such as, for example, pyrrolidine or piperidine. In the case of cyclic secondary amines the products are believed to be formed by a mechanism involving ring-opening and condensation with the elimination of ammonia. Thus, for example, in the case of pyrrolidine the reaction may be represented as follows:—

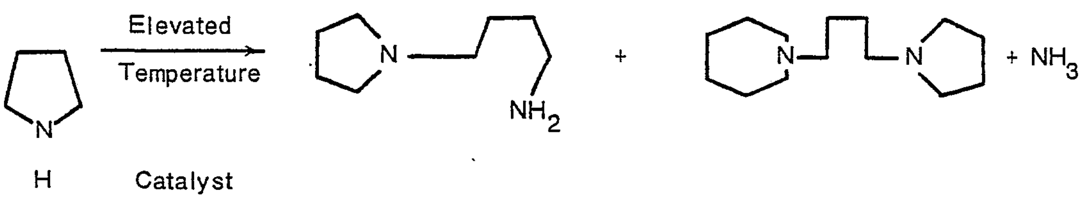


(I) (II)

The process may suitably be carried out at a temperature in the range 150 to 275°C, preferably in the range 175 to 250°C. The reaction time in a batch process in which the amine and catalyst are charged to a reactor and maintained at elevated temperature may suitably be in the range from 5 to 120 hours, preferably from 30 to 120 hours.

(vii) the production of polyphenylenemethylene by reacting benzyl alcohol in the presence as catalyst of a cation-exchangeable layered clay in which the exchangeable cation is a metal cation.

The polymer contains repeat units of the type:

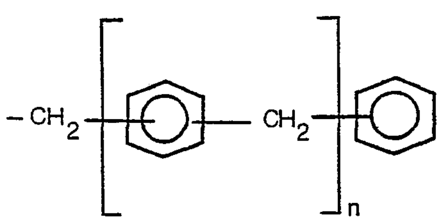

in which the average value of n is 10.

(vii) the production of thioethers by reacting alkanthiols at elevated temperature in the presence of a cation-exchangeable layered clay in which the exchangeable cations is a metal cation.

(ix) the production of alkyl aromatic compounds by reacting one or more olefins, e.g. the production of ethylbenzene by reacting benzene with ethylene, and isopropylbenzene by reacting benzene with propylene, at elevated temperatures suitably up to 250°C preferably in the range 175 to 250°C and elevated temperature and atmospheric or elevated pressure.

(xi) the production of alcohols by the hydration of olefins at elevated temperature and pressure where the water reactant consumed is more than the intercalated water contained in the clay.

The invention will now be illustrated by reference to the following Examples.

All analytical results were determined using gas chromatography and the identity of the products were confirmed by comparison with authentic materials, mass spectroscopy or nuclear magnetic resonance spectroscopy.

PREPARATION OF METAL CATION-EXCHANGED LAYERED CLAY

Finally divided sodium bentonite was cation exchanged with 0.5 M aqueous solutions of appropriate salts for periods of approximately 24 hours (the chromium salt employed was $Cr_2(SO_4)_3$; the aluminium salt employed was $Al_2(SO_4)_3.16H_2O$). The solutions were mechanically stirred during the first 2 hours of a period of 24 hours, after which the solid was washed repeatedly with deionised water until the excess cations had been removed. Surplus liquid was removed from the solid using a teat-pipette prior to drying in a vacuum oven at 60°C. When the clay was visibly dry it was ground until it passed 140 BSS mesh sieve. The cation-exchangeable clay was then equilibrated over granular anhydrous calcium chloride in a dessicator for a minimum period of 24 hours.

PRODUCTION OF ESTERS BY REACTION OF AN OLEFIN WITH A CARBOXYLIC ACID IN THE LIQUID PHASE

Example 1

$Cr^{3+}$-exchanged bentonite (0.5 g), hex-1-ene (5 ml) and acetic acid (1.5 ml) were placed in a standard steel reactor of capacity 20 ml. The reactor was closed by a screw cap provided with an O-ring seal and immersed up to the screw cap in a silicone oil bath which was maintained at 200°C. After 4 hours the reactor was removed from the bath, cooled and its contents analysed. The results in terms of the weight percentage of individual products in the product mixture (rounded to the nearest whole number) are given in Table 1.

Example 2

Example 1 was repeated except that the $Cr^{3+}$-exchanged bentonite was replaced by $Al^{3+}$-exchanged bentonite and the acetic acid was replaced by propionic acid.

Example 3

Example 1 was repeated except that the $Cr^{3+}$-exchanged bentonite was replaced by $Al^{3+}$-exchanged bentonite and acetic acid was replaced by isobutyric acid.

Example 4

Example 1 was repeated except that hex-1-ene was replaced by hept-1-ene.

Example 5

Example 1 was repeated except that hex-1-ene was replaced by oct-1-ene.

Example 6

Example 1 was repeated except that the $Cr^{3+}$-exchanged bentonite was replaced by $Al^{3+}$-exchanged bentonite and hex-1-ene was replaced by 4-methylpent-1-ene.

Example 7

Example 1 was repeated except that the $Cr^{3+}$-exchanged bentonite was replaced by $Al^{3+}$-exchanged bentonite and hex-1-ene was replaced by hex-2-ene.

The results of Examples 1 to 7 are given in Table 1.

Example 8

Acetic acid (80 g) and $Al^{3+}$-exchanged Wyoming Bentonite catalyst (10 g, 9.4% wt Al) were charged to a 100 ml stirred autoclave. The autoclave was charged with ethylene to a pressure of 55 bar (ethylene:acetic acid mole ratio 2:1). The temperature of the autoclave was raised to 250°C and maintained at this temperature for 2.5 hr.

At the end of the reaction period the autoclave was cooled, vented and the product analysed. The yield of ethyl acetate was 25% and the selectivity to ethyl acetate was greater than 99%, both yield and selectivity being based on acetic acid.

Example 9

Example 8 was repeated except that the autoclave was pressurised to 150 bar with ethylene and the temperature was reduced to 200°C. The yield of ethyl acetate was 35% and the selectivity greater than 99%.

Example 10

Example 8 was repeated except that the temperature was reduced to 200°C. The yield of ethyl acetate was 18% at a selectivity greater than 99%.

Example 11

Example 10 was repeated except that the catalyst was activated before use by heating in air at 200°C.

The yield of ethyl acetate was increased from 18% to 28%.

Example 12

Example 11 was repeated except that the catalyst heat treatment was carried out above 300°C. This treatment led to substantial catalyst deactivation. The yield of ethyl acetate was about 4% compared with 18% in Example 10.

Example 13

Example 10 was repeated using less catalyst (3 g).

The yield of ethyl acetate was reduced and this was also found to be the case even when the reaction time was increased.

PRODUCTION OF ETHERS BY REACTING AN ALKANOL WITH AN OLEFIN

Example 14

The procedure described in Example 1 was followed except that the $Cr^{3+}$-exchanged bentonite was replaced by $Al^{3+}$-exchanged bentonite and the hex-1-ene/acetic acid was replaced by a 50:50 v/v mixture (5 ml) of hexan-1-ol and hex-1-ene. Analysis of the product mixture showed:—

| | wt % of product mixture |
|---|---|
| Hexene | 53 |
| Hexanol | 8 |
| 1,1-ether | 9 |
| 1,2 + 1,3-ethers | 4 |

Example 15

5 g of the dry aluminium ion-exchanged Wyoming bentonite, hex-1-ene (25 g) and methanol (19 g) were sealed in a Baskerville 100 ml stainless steel autoclave fitted with a stirrer. The autoclave was heated at 200°C for 2.5 hours, and then cooled. The liquid products (32.0 g, 73% weight recovered) were recovered and shown to contain 2-methoxyhexane (10.2%) and dimethyl ether (4.9%) as the two major new products. The product percentages are based on peak areas shown on a flame ionisation gas chromatograph. The gaseous products were not examined.

Example 16

5 g of the dry aluminium ion-exchanged Wyoming bentonite and methanol (19 g) were cooled to —20°C in the bottom-half of a Baskerville 100 ml stainless steel autoclave. But-1-ene (ca 20 ml of condensed liquid in a cardice cold trap) was added and the autoclave sealed. The autoclave was flushed with nitrogen and stirred at 200°C for 2.5 hours, and allowed to cool. The liquid products (7 g, 10% weight recovered) were recovered and shown to contain 2-methoxybutane (34.7%), and $C_4$ dimers (56.2%) as the two major new peaks. The product percentages are based on peak areas shown on a flame ionisation gas chromatograph. The gaseous products were not examine.

PRODUCTION OF ETHERS BY REACTION OF A PRIMARY OR SECONDARY ALIPHATIC ALCOHOL OR A POLYOL

Example 17

$Al^{3+}$-exchanged bentonite (0.5 g) and butan-1-ol were placed in a standard steel reactor of capacity 20 ml. The reactor was closed by a screw cap provided with an O-ring seal and immersed up to the screw cap in a silicone oil bath maintained at 200°C. After 4 hours the reactor was removed from the bath, cooled and its contents analysed.

Example 18

Example 17 was repeated except that butan-1-ol was replaced by pentan-1-ol.

Example 19

Example 17 was repeated except that butan-1-ol was replaced by hexan-1-ol.

Example 20

Example 17 was repeated except that butan-1-ol was replaced by heptan-1-ol.

Example 21

Example 17 was repeated except that butan-1-ol was replaced by octan-1-ol.

Example 22

Example 17 was repeated except that butan-1-ol was replaced by 3-methylbutan-1-ol.

Example 23

Example 17 was repeated except that butan-1-ol was replaced by 3-methylpentan-1-ol.

Example 24

Example 17 was repeated except that butan-1-ol was replaced by propan-2-ol.

Example 25

Example 17 was repeated except that butan-1-ol was replaced by butan-2-ol.

Example 26

Example 17 was repeated except that butan-1-ol was replaced by pentan-2-ol.

Example 27

Example 17 was repeated except that butan-1-ol was replaced by hexan-2-ol.

Example 28

Example 17 was repeated except that butan-1-ol was replaced by 2-methylbutan-2-ol. This example is included to illustrate the fact that tertiary alkanols yield only alkene and alkene dimers. Identical results have been obtained from 2-methylpropan-2-ol; 2-methyl-pentan-2-ol and 3-methylpentan-3-ol.

The results of Examples 17 to 28 in terms of the wt. % of individual products in the product mixture (rounded to the nearest whole number) are given in Table 2.

Example 29

Example 17 was repeated except that butan-1-ol was replaced by diethylene glycol.
Analysis of the product showed:—

| | wt % product mixture |
|---|---|
| Unreacted diethylene glycol | 57 |
| Dioxan | 20 |
| Ethylene glycol | 8 |
| Triethylene glycol | 14 |
| Others | 1 |

Example 30

Example 17 was repeated except that butan-1-ol was replaced by butan-1-thiol.
Analysis of the product showed:—

| | wt % product mixture |
|---|---|
| Unreacted thiol | 51 |
| Thioether | 30 |
| Disulphide | 15 |
| Others | 4 |

Example 31

$Cr^{3+}$-exchanged bentonite (0.5 g) and benzylamine (5 ml) were placed in the standard steel reactor described in Example 1. The reactor was heated for 12 hours in a silicone oil bath maintained at 200°C. It was then cooled and its contents analysed.

Example 32

Example 31 was repeated except that benzylamine was replaced by cyclohexylamine and the reaction time was extended to 9 hours.

Example 33

Example 31 was repeated except that the $Cr^{3+}$-exchanged bentonite was replaced by $Al^{3+}$-exchanged bentonite, benzylamine was replaced by hexan-1-amine and the reaction time was extended to 22 hours.

The results of Example 31 to 33 in terms of the wt. % of individual products in the product mixture (rounded to the nearest whole number) are given in Table 3.

Example 34

Example 31 was repeated except that the $Cr^{3+}$-exchanged bentonite was replaced by $Al^{3+}$-exchanged bentonite, benzylamine was replaced by pyrrolidone and the reaction time was extended to 36 hours.

Analysis of the product showed:—

| | wt. % of product mixture |
|---|---|
| Unreacted pyrrolidine | 33 |
| 4-(1-pyrrolidyl)butanamine | 40 |
| 1,4-di-(1-pyrrolidyl)butane | 26 |
| Others | 1 |

Example 35

Ethylene oxide (5 ml) and aluminium-exchanged bentonite (0.5 g) were placed in a steel reactor of capacity 20 ml. The reactor was sealed with a screw cap and heated to 110°C. After 6 hours the reactor was covered and the contents analysed. The product contained 1,4-dioxan (68%) and 2 methyl-1,3-dioxolane (16%) together with unchanged reactants and some unidentified minor components.

Example 36

Ethylene oxide (5 ml), acetic acid (2.5 ml) and aluminium-exchanged bentonite (0.5 g) were placed in a steel reactor of capacity 20 ml. The reactor was sealed with a screw cap and heated to 100°C. After 60 minutes the reactor was cooled and the contents analysed. The product contained 1,4-dioxan (35%), ethylene glycol diacetate (17%), 2-hydroxyethylacetate (11%), 2-methyl-1,3-dioxolane (7%) and but-2-en-1-al (3%) together with unchanged reactants and some unidentified minor components.

Example 37

Ethylene oxide (2.5 ml) ethanol (2.5 ml) and aluminium exchanged bentonite (0.5 g) were in a steel reactor of capacity 20 ml. The reactor was sealed with a screw cap and heated to 110°C. After 4 hours the reactor was cooled and the contents analysed. The product contained diethyl ether (2%), ethylene glycol diethyl ether (1%), and diethylene glycol mono-ethylether (21%) together with unchanged reactants and some unidentified minor components.

Example 38

Propylene oxide (5 ml) and aluminium-exchanged bentonite (0.5 g) were placed in a steel reactor of capacity 20 ml. The reactor was sealed with a screw cap and heated to 200°C. After 8 hours the reactor was cooled and the contents analysed. The product contained dimethyl-1,4-dioxane (4%), 2-methyl-2-pentanol (35%) and 1,3-dioxolanes (16%) together with unchanged reactant and some unidentified minor components.

Example 39

Acetaldehyde (5 ml) and aluminium-exchanged bentonite (0.5 g) were placed in a steel reactor of capacity 20 ml. The reactor was sealed and heated at 110°C. After 8 hours the reactor was cooled and the contents analysed. The product contained but-2-en-1-al (24%) together with unchanged reactant and some unidentified minor components.

TABLE 1

| Example | Catalyst | Alkene | Acid | wt. % in product mixture | | | |
|---|---|---|---|---|---|---|---|
| | | | | Unreacted alkene | Unreacted acid | Total esters | Alkene dimers |
| 1 | $Cr^{3+}$-bentonite | Hex-1-ene | acetic | 41 | 36 | 16 | 7 |
| 2 | $Al^{3+}$-bentonite | Hex-1-ene | propionic | 31 | 33 | 11 | 25 |
| 3 | $Al^{3+}$-bentonite | Hex-1-ene | isobutyric | 45 | 38 | 6 | 11 |
| 4 | $Cr^{3+}$-bentonite | Hept-1-ene | acetic | 47 | 37 | 11 | 5 |
| 5 | $Cr^{3+}$-bentonite | Oct-1-ene | acetic | 60 | 28 | 12 | 0 |
| 6 | $Al^{3+}$-bentonite | 4MePent-1-ene | acetic | 49 | 22 | 12 | 17 |
| 7 | $Al^{3+}$-bentonite | hex-2-ene | acetic | 50 | 20 | 21 | 7 |

TABLE 2

| Example | Alkanol | wt. % of reaction product | | | | | |
|---|---|---|---|---|---|---|---|
| | | Unreacted alkene | 2,2-dialkyl ether | 1,1-dialkyl ether | 1,2-dialkyl ethers | Alkenes | Alkene dimers |
| 17 | Butan-1-ol | 39 | — | 51 | 4 | 6* | 1 |
| 18 | Pentan-1-ol | 42 | — | 38 | 4 | 11 | 6 |
| 19 | Hexan-1-ol | 30 | — | 45 | 4 | 16 | 4 |
| 20 | Heptan-1-ol | 42 | — | 34 | 2 | 15 | 6 |
| 21 | Octan-1-ol | 38 | — | 36 | — | 20 | 6 |
| 22 | 3-Mebutan-1-ol | 50 | — | 35 | — | 4 | 12 |
| 23 | 3-Mepentan-1-ol | 44 | — | 25 | — | 15 | 16 |
| 24 | Propan-2-ol | 49 | 47 | — | — | 3* | 1 |
| 25 | Butan-2-ol** | 35 | 32 | — | — | 25* | 1 |
| 26 | Pentan-2-ol | 9 | 6 | — | — | 85 | 1 |
| 27 | Hexan-2-ol | 3 | 1 | — | — | 95 | 2 |
| 28 | 2-Mebutan-2-ol | 3 | — | — | — | 90 | 7 |

* Due to loss of gaseous alkenes on sampling these figures are minimised, hence all others in the relevant line of the Table are maxima.
** The 7% deficit in this analysis is butan-2-one which appears to be an impurity in the reactant.

TABLE 3

| Example | Layered Clay | Amine | wt. % of product mixture | | |
|---|---|---|---|---|---|
| | | | Unreacted amine | Secondary amine | Others |
| 31 | $Cr^{3+}$-bentonite | Benzylamine | 71 | 21 | 5 |
| 32 | $Cr^{3+}$-bentonite | Cyclohexylamine | 81 | 18 | 1 |
| 33 | $Al^{3+}$-bentonite | Hexan-1-amine | 96 | 2 | 2 |

## Claims

1. A process for carrying out a proton-catalysed organic reaction in which intercalated water is not one of the stoichiometric reactants employing as catalyst a metal cation-exchangeable material characterised in that as the cation-exchangeable material there is used a layered clay in which the exchangeable cation is a metal cation.

2. A process according to claim 1 wherein the layered clay is a bentonite.

3. A process according to either claim 1 or claim 2 wherein some or all of the exchangeable cations are exchanged with cations of one or more of the metals chromium, aluminium, cobalt, nickel, iron, copper and vanadium.

4. A process according to claim 3 wherein the metal is chromium or aluminium.

5. A process according to any one of the preceding claims wherein the proton-catalysed organic reaction is the production of esters by reacting one or more olefins with one or more carboxylic acids under reaction conditions which result in the formation of an ester.

6. A process according to claim 5 wherein the olefin is ethylene, propylene, a butene, a pentene, a hexene or cyclohexene.

7. A process according to either one of claims 5 or 6 wherein the carboxylic acid is formic acid, acetic acid, propionic acid, butyric acid or a phthalic acid.

8. A process according to any one of claims 5 to 7 wherein the olefin is ethylene, the carboxylic acid is acetic acid and the ester is ethyl acetate.

9. A process according to any one of claims 5 to 8 wherein the olefin is contacted with the carboxylic acid in the liquid phase at a temperature in the range from 100 to 300°C and a pressure which maintains a liquid phase at the reaction temperature.

10. A process according to any one of claims 5 to 8 wherein the olefin is contacted with the carboxylic acid in the vapour phase at a temperature in the range from 120 to 180°C and a residence time in the range from 10 to 60 seconds.

11. A process according to any one of claims 1 to 4 wherein the proton-catalysed organic reaction is the production of ethers by reacting at a temperature above 100°C one or more alcohols with one or more olefins having the structure:—

$$R^1CH = CHR^2$$

wherein $R^1$ and $R^2$ are independently H, alkyl or aryl or are bonded together to form a ring.

12. A process according to claim 11 wherein the alcohol is methanol, ethanol, a propanol, a butanol, a pentanol or a hexanol.

13. A process according to either one of claims 11 or 12 wherein the olefin is ethylene, propylene, a butene, a pentene or a hexene.

14. A process according to any one of claims 11 to 13 wherein the alcohol is contacted with the olefin in the liquid phase.

15. A process according to any one of the previous claims wherein the catalyst is activated before use by heating in air at a temperature in the range 80 to 220°C.

16. A process according to any one of claims 1 to 4 wherein the proton-catalysed reaction is the production of ethers by reacting at elevated temperature and a pressure such that the reactants are maintained in the liquid phase a primary or secondary aliphatic alcohol or a polyol.

17. A process according to any one of claims 1 to 4 wherein the proton-catalysed reaction is the production of an alkyl aromatic compound by reacting one or more aromatic compounds with one or more olefins.

18. A process according to claim 17 wherein the aromatic compound is benzene, the olefin is ethylene and the alkyl aromatic compound is ethyl benzene.

19. A process according to claim 17 wherein the aromatic compound is benzene, the olefin is propylene and the alkyl aromatic compound is isopropylbenzene.

20. A process according to any one of claims 1 to 4 wherein the proton-catalysed reaction is the production of ethers by reacting one or more epoxides.

21. A process according to any one of claims 1 to 4 wherein the proton-catalysed reaction is the production of esters by reacting one or more epoxides with one or more carboxylic acids.

22. A process according to any one of claims 1 to 4 wherein the proton-catalysed reaction is the production of ethers by reacting one or more epoxides with one or more alcohols, polyols or polysaccharides.

23. A process according to any one of claims 1 to 3 wherein the proton-catalysed reaction is the production of esters by reacting one or more alcohols with one or more carboxylic acids.

24. A process according to any one of claims 1 to 4 wherein the proton-catalysed reaction is the hydration of olefins.

**Patentansprüche**

1. Verfahren zur Durchführung einer Protonen-katalysierten organischen Reaktion, in welcher eingelagertes Wasser nicht einer der stöchiometrischen Reaktanten ist, wobei als Katalysator ein Metallkation-austauschbares Material verwendet wird, dadurch gekennzeichnet, daß als Kationaustauschbares Material ein Schichtton verwendet wird, in welchem das austauschbare Kation ein Metallkation ist.

2. Verfahren gemäß Anspruch 1 worin der Schichtton ein Bentonit ist.

3. Verfahren gemäß Anspruch 1 oder 2 worin einige oder alle der austauschbaren Kationen ausgetauscht sind mit Kationen eines oder mehrerer der Metalle Chrom, Aluminium, Kobalt, Nickel, Eisen, Kupfer und Vanadium.

4. Verfahren gemäß Anspruch 3 worin das Metall Chrom oder Aluminium ist.

5. Verfahren gemäß einem der vorgehenden Ansprüche worin die Protonen-katalysierte organische Reaktion die Herstellung von Estern durch Umsetzung von einem oder mehreren Olefinen mit einer oder mehreren Carbonsäuren unter Reaktionsbedingungen, die zu einer Esterbildung führen, ist.

6. Verfahren gemäß Anspruch 5 worin das Olefin Äthylen, Propylen, ein Buten, ein Penten, ein Hexen oder Cyclohexen ist.

7. Verfahren gemäß einem der Ansprüche 5 oder 6 worin die Carbonsäure Ameisensäure, Essigsäure, Propionsäure, Buttersäure oder Phthalsäure ist.

8. Verfahren gemäß einem der Ansprüche 5 oder 7 worin das Olefin Äthylen, die Carbonsäure Essigsäure und der Ester Äthylacetat sind.

9. Verfahren gemäß einem der Ansprüche 5 bis 8 worin das Olefin mit der Carbonsäure in der flüssigen Phase bei einer Temperatur im Bereich von 100 bis 300°C und einem Druck, der eine flüssige Phase bei der Reaktionstemperatur aufrechterhält, in Berührung gebracht wird.

10. Verfahren gemäß einem der Ansprüche 5 bis 8, worin das Olefin mit der Carbonsäure in der Dampfphase bei einer Temperatur im Bereich von 120 bis 180°C und einer Verweilzeit im Bereich von 10 bis 16 Sekunden in Berührung gebracht wird.

11. Verfahren gemäß einem der Ansprüche 1 bis 4 worin die Protonen-katalysierte organische Reaktion die Herstellung von Äthern durch Umsetzung bei einer Temperatur oberhalb 100°C von einem oder mehreren Alkoholen mit einem oder mehreren Olefinen der Struktur:

$$R^1CH = CHR^2$$

worin $R^1$ und $R^2$ unabhängig Wasserstoff, Alkyl oder Aryl bedeuten oder unter Bildung eines Ringes miteinander verbunden sind, ist.

12. Verfahren gemäß Anspruch 11, worin der Alkohol Methanol, Äthanol, ein Propanol, ein Butanol, ein Pentanol oder ein Hexanol ist.

13. Verfahren gemäß einem der Ansprüche 11 oder 12 worin das Olefin Äthylen, Propylen, ein Buten, ein Penten oder ein Hexen ist.

14. Verfahren gemäß einem der Ansprüche 11 bis 13 worin der Alkohol mit dem Olefin in der flüssigen Phase in Berührung gebracht wird.

15. Verfahren gemäß einem der vorgehenden Ansprüche worin der Katalysator vor Verwendung aktiviert wird durch Erhitzen in Luft auf eine Temperatur im Bereich von 80 bis 220°C.

16. Verfahren gemäß einem der Ansprüche 1 bis 4 worin die Protonen-katalysierte Reaktion die Herstellung von Äthern durch Umsetzung eines primären oder sekundären aliphatischen Alkohols oder eines Polyols bei erhöhter Temperatur und einem Druck, der die Reaktanten in flüssiger Phase hält, ist.

17. Verfahren gemäß einem der Ansprüche 1 bis 4 worin die Protonen-katalysierte Reaktion die Herstellung einer alkyl-aromatischen Verbindung ist durch Umsetzung von einer oder mehreren aromatischen Verbindungen mit einem oder mehreren Olefinen.

18. Verfahren gemäß Anspruch 17 worin die aromatische Verbindung Benzol, das Olefin Äthylen und die alkyl-aromatische Verbindung Äthylbenzol sind.

19. Verfahren gemäß Anspruch 17 worin die aromatische Verbindung Benzol, das Olefin Propylen und die Alkyl-aromatische Verbindung Isopropylbenzol sind.

20. Verfahren gemäß einem der Ansprüche 1 bis 4 worin die Protonen-katalysierte Reaktion die Herstellung von Äthern durch Umsetzung von einem oder mehreren Epoxyden ist.

21. Verfahren gemäß einem der Ansprüche 1 bis 4 worin die Protonen-katalysierte Reaktion die Herstellung von Estern durch Umsetzung von einem oder mehreren Epoxyden mit einer oder mehreren Carbonsäuren ist.

22. Verfahren gemäß einem der Ansprüche 1 bis 4 worin die Protonen-katalysierte Reaktion die Herstellung von Äthern durch Umsetzung von einem oder mehreren Epoxyden mit einem oder mehreren Alkoholen, Polyolen oder Polysacchariden ist.

23. Verfahren gemäß einem der Ansprüche 1 bis 3 worin die Protonen-katalysierte Reaktion die Herstellung von Estern durch Umsetzung von einem oder mehreren Alkoholen mit einer oder mehreren Carbonsäuren ist.

24. Verfahren gemäß einem der Ansprüche 1 bis 4 worin die Protonen-katalysierte Reaktion die Hydratisierung von Olefinen ist.

## Revendications

1. Procédé pour effectuer une réaction organique, catalysée par des protons, dans laquelle l'eau intercalée ne constitue pas l'un des corps stoechiométriques destinés à réagir, et utilisant comme catalyseur une matière comportant un cation de métal pouvant être échangé, procédé caractérisé en ce que l'on utilise, comme matière comportant un cation pouvant être échangé, une argile litée dans laquelle le cation pouvant être échangé est un cation de métal.

2. Procédé selon la revendication 1, dans lequel l'argile litée est une bentonite.

3. Procédé selon l'une des revendications 1 ou 2, dans lequel quelques-uns ou tous les cations pouvant être échangés sont remplacés par échange avec des cations d'un ou plusieurs des métaux

chrome, aluminium, cobalt, nickel, fer, cuivre et vanadium.

4. Procédé selon la revendication 3, dans lequel le métal est le chrome ou l'aluminium.

5. Procédé selon l'une quelconque des revendications précédentes, dans lequel la réaction organique catalysée par des protons est la production d'esters par réaction d'une ou plusieurs oléfines avec un ou plusieurs acides carboxyliques dans des conditions de réaction aboutissant à la formation d'un ester.

6. Procédé selon la revendication 5, dans lequel l'oléfine est l'éthylène, le propylène, un butène, un pentène, un hexène ou du cyclohexène.

7. Procédé selon l'une quelconque des revendications 5 ou 6, dans lequel l'acide carboxylique est l'acide formique, l'acide acétique, l'acide propionique, l'acide butyrique ou un acide phtalique.

8. Procédé selon l'un quelconque des revendications 5 ou 7, dans lequel l'oléfine est l'éthylène, l'acide carboxylique est l'acide acétique, et l'ester est l'acétate d'éthyle.

9. Procédé selon l'une quelconque des revendications 5 à 8, dans lequel on met l'oléfine en contact avec l'acide carboxylique en phase liquid à une température comprise entre 100 et 300°C et à une pression qui maintient une phase liquid à la température de réaction.

10. Procédé selon l'une quelconque des revendications 5 à 8, dans lequel on met l'oléfine en contact avec l'acide carboxylique en phase vapeur à une température comprise entre 120 et 180°C et pendant un temps de séjour compris entre 10 et 60 secondes.

11. Procédé selon l'une quelconque des revendications 1 à 4, dans lequel la réaction organique catalysée par des protons est la production d'éthers par réaction, à une température supérieure à 100°C, d'un ou plusieurs alcools avec une ou plusieurs oléfines ayant la structure

$$R^1CH = CHR^2$$

dans laquelle $R^1$ et $R^2$ sont indépendamment H, un groupe alkyle ou aryle ou sont reliés ensemble pour former un noyau.

12. Procédé selon la revendication 11, dans lequel l'alcool est le méthanol, l'éthanol, un propanol, un butanol, un pentanol ou un hexanol.

13. Procédé selon l'une des revendications 11 ou 12, dans lequel l'oléfine est l'éthylène, le propylène, un butène, un pentène ou un hexène.

14. Procédé selon l'une quelconque des revendications 11 à 13, dans lequel on met l'alcool en contact avec l'oléfine en phase liquide.

15. Procédé selon l'une quelconque des revendications précédentes, dans lequel on active le catalyseur, avant de l'utiliser, en le chauffant à l'air à une température comprise entre 80 et 220°C.

16. Procédé selon l'une quelconque des revendications 1 à 4, dans lequel la réaction catalysée par des protons est la production d'éthers, par mise en réaction, à température élevée et à une pression telle que les corps destinés à réagir sont maintenus en phase liquide, d'un alcool aliphatique primaire ou secondaire ou d'un polyol.

17. Procédé selon l'une quelconque des revendications 1 à 4, dans lequel la réaction catalysée par des protons est la production d'un composé alkyl aromatique par la réaction d'un ou plusieurs composés aromatiques avec une ou plusieurs oléfines.

18. Procédé selon la revendication 17, dans lequel le composé aromatique est le benzène, l'oléfine est l'éthylène, et le composé alkyl aromatique est l'éthyl benzène.

19. Procédé selon la revendication 17, dans lequel le composé aromatique est le benzène, l'oléfine est le propylène, et le composé alkyl aromatique est l'isopropylbenzène.

20. Procédé selon l'une quelconque des revendications 1 à 4, dans lequel la réaction catalysée par des protons est la production d'éthers par mise en réaction d'un ou plusieurs époxydes.

21. Procédé selon l'une quelconque des revendications 1 à 4, dans lequel la réaction catalysée par des protons est la production d'esters par mise en réaction d'un ou plusieurs époxydes avec un ou plusieurs acides carboxyliques.

22. Procédé selon l'une quelconque des revendications 1 à 4, dans lequel la réaction catalysée par des protons est la production d'éthers par la mise en réaction d'un ou plusieurs époxydes avec un ou plusieurs alcools, polyols ou polysaccharides.

23. Procédé selon l'une quelconque des revendications 1 à 3, dans lequel la réaction catalysée par des protons est la production d'esters par mise en réaction d'un ou plusieurs alcools avec un ou plusieurs acides carboxyliques.

24. Procédé selon l'une quelconque des revendications 1 à 4, dans lequel la réaction catalysée par des protons est l'hydratatoin d'oléfines.